# EUROPEAN PATENT SPECIFICATION

(11) **EP 0 824 906 B1**
(45) Date of publication and mention of the grant of the patent: **25.02.2004**
(21) Application number: 97306172.4
(22) Date of filing: 13.08.1997
(51) Int. Cl.: A61F 13/15, A61F 13/56

(54) **Disposable diaper**
Wegwerfwindel
Couche-culotte jetable

(30) Priority: 19.08.1996 JP 21737796; 19.11.1996 JP 30810896; 26.12.1996 JP 34846596
(43) Date of publication of application: 25.02.1998
(60) Divisional application: 01203078.9
(73) Proprietor: UNI-CHARM CORPORATION, Kawanoe-shi Ehime-ken (JP)
(72) Inventor: Fujioka, Yoshihisa, Mito-gun, Kagawa-ken (JP); Yamaki, Rumi, Kawanoe-shi, Ehime-ken (JP); Ono, Yoshio, Kawanoe-shi, Ehime-ken (JP)
(74) Representative: Parry, Christopher Stephen

(56) References cited:
- EP-A- 0 641 552
- EP-A- 0 758 543
- WO-A-90/04374
- WO-A-91/09580
- WO-A-95/06451
- GB-A- 2 080 093
- GB-A- 2 262 873
- US-A- 4 351 340
- US-A- 4 680 030
- US-A- 4 775 375
- US-A- 4 911 702
- US-A- 5 382 246

## Description

The present invention relates to a disposable diaper which fits the body of a wearer and is designed to prevent the positional shift of a protector against incontinence and the side oozing of for example urine particularly when such protector against incontinence is used.

Disposable diaper comprises a back sheet, a top sheet and an absorption core interposed between the two sheets. The back sheet facing outward comprises a resin sheet, liquid non-permeable but finely air permeable so as to prevent the outside oozing of urine. Furthermore, the top sheet facing toward the side of skin is therefore in direct contact to skin and works as the side to be spotted with urine. Hence, the top sheet is formed of a material gentle to skin, such as non-woven fabric and soft porous sheets, both liquid permeable, so that the top sheet might be permeated with fluids such as urine into the absorption core. As the absorption core, additionally, use is made of a crushed pulp or a mixture of a crushed pulp and super absorbent polymers.

So as to fit the body outline of a wearer, the absorption core is preliminarily cut and molded for example as a sandglass-like form. After molding, the absorption core is interposed between the back sheet and the top sheet. The back sheet and the top sheet both are of a shape similar to the shape of the absorption core but with a larger outline than the absorption core. At both the ends thereof where no absorption core is present, these back sheet and top sheet are bonded together by means of a hot-melt adhesive.

The back waist region of disposable diaper is attached to the dorsal area of a wearer; the crotch region thereof is attached to the crotch thereof; and the front waist region is attached to the abdominal area thereof. By drawing forward the flaps of the back waist region which flaps elongate in the width direction of the diapers to overlay the flaps over the front waist region, the front waist region and the flaps are fastened together. Thus, the diapers are completely worn.

Generally, flat elastic braids capable of elongating toward the longitudinal direction (lengthwise direction) of the diapers are partially bonded, at their elongated state between the back sheet and the top sheet, to the end parts of the crotch region in the crosswise direction (width direction) by means of a hot-melt adhesive, whereby gathering is formed at the thigh parts of the diapers.

Disposable diaper for adults is far larger than disposable diaper for infants and toddlers, requiring a larger volume of raw materials for one diaper piece and thus costing high per unit price. Therefore, it is not economical to exchange the diapers for adults on each excretion of urine. Accordingly, a protector against incontinence is generally used in combination of the diapers. After urine excretion, then, only the protector against incontinence is to be exchanged.

Such protector against incontinence is formed of a lamination body in a sheet-like form, comprising an outer sheet comprising for example a liquid non-permeable resin sheet, an inner sheet comprising for example a non-woven fabric, and an absorption core interposed between the two sheets. At the ends in the lamination body in the width direction and the longitudinal direction where no absorption core is present, the outer sheet and the inner sheet are bonded together by means of a hot-melt adhesive. To the' ends in the width direction, additionally, an elastic member such as flat elastic braids are partially bonded at its elongated state between the outer sheet and the inner sheet. The elastic member forms gathering.

The lamination body in the sheet-like form is preliminarily molded in the shape of a cone so that the center of the lamination body in the sheet-like form might be the peak when the protector against incontinence is worn. Through an adhesive part, both the ends of the lamination body are bonded together.

Disposable diaper should be used in such a manner that when a male wearer wears such protector against incontinence, the top sheet of diapers adheres to the adhesive part in the crotch region of the protector to prevent the shift of the protector in the diaper.

A protector against incontinence of the same structure may be used as a protector against incontinence for females. While the longitudinal direction of the lamination body should be aligned along the longitudinal direction of a disposable diaper, the outer sheet of the protector should face the top sheet of the disposable diaper to overlay the protector over the crotch region of the diaper. Then, the adhesive part of the protector against incontinence adheres to the top sheet of the diaper to prevent the shift of the protector on the diaper. In such manner, the protector is used.

These diapers are preliminarily molded as a sandglass-like form, wherein the width of the crotch region is narrower than the widths of the front waist region and the back waist region. Because urine is mainly absorbed in the crotch region, however, the crotch region should be at some width dimension so as to absorb urine. Therefore, the crotch region is formed at a larger width than the width of the crotch of humans.

At a state of such diaper worn, the crotch region expands in a bag-like shape because the crotch region is pressed by the thigh parts of a wearer from both the sides in the width direction, so that a space is formed between the crotch of the wearer and the diaper. When the diaper is used in combination with a protector against incontinence, therefore, the protector is positioned in the expanding part in front of the crotch region of the diaper. Then, the protector against incontinence is not pressed against the body. When the wearer makes a motion, therefore, the protector so readily makes a positional shift on the diaper that the protector cannot effectively absorb urine.

In the border region between the front waist region and the crotch region of a diaper, in particular, the individual dimensions of these regions in the width directions are prominently different from each other. When the crotch region is pressed by the thigh parts, therefore, the side of the front waist region essentially expands in the border region between the crotch region and the front waist region. The protector against incontinence for males is mounted at a position closer to the front waist region in the border region between the front waist region and the crotch region. When the front waist region expands as has been described above, therefore, the force of the diaper to press the protector is weakened especially for males, to readily cause the positional sift of the protector against incontinence.

When the diaper is directly worn without any protector against incontinence, excretion such as urine and feces might by chance ooze from the space between the diaper and the body of the wearer sideward. Additionally, excretion cannot be retained at a given position of the diaper, which may make the wearer feel unpleasant feeling. In this case, also, a part closer to the side of the front waist region in the border region between the front waist region and the back waist region mainly expands readily, so that excretion easily oozes out from the part.

US 4,680,030 discloses a disposable diaper comprising a liquid non-permeable back sheet, a liquid permeable top sheet and an absorption core interposed between the two sheets, having a front waist region to be applied to the abdominal area of a wearer, a back waist region to be applied to the dorsal area thereof, and a crotch region to be applied to the crotch. Two first retaining members are arranged at an interval in the longitudinal direction on the surface of the front waist region, provided that the direction from the front waist region through the crotch region to the back waist region is defined as the longitudinal direction, and two second retaining members to fasten to the first retaining members are arranged at an interval in the longitudinal direction on both the ends of the back waist region to be overlaid over the front waist region.

When the direction from the front waist region through the crotch region to the back waist region is defined as the longitudinal direction of the diaper, conventionally, the aligning pitch of the retaining fasteners arranged on the front waist region in the longitudinal direction has been almost the same as the aligning pitch of the retaining fasteners arranged on the flaps of the back waist region in the longitudinal direction. When the flaps of the back waist region are overlaid over the surface of the front waist region to fasten the individual retaining fasteners together, therefore, the inner space of the diaper from a cylindrical shape. The dimension of the inner diameter (diameter size) of the cylinder is kept at almost the same value all through the longitudinal direction of the diaper.

However, the outer size of the truck part (waist size) of humans is generally smaller than the outer size of the hip part thereof. Therefore, the inner size of the diaper with the retaining fasteners fastened together is adjusted to the outer size of the hip part, so that some space is formed between the diaper and the trunk (waist) part of the body. When the retaining fasteners are fastened together, therefore, the diaper cannot fit the waist, causing insufficient fastening of the diaper around the waist, which possibly causes certain positional shift of the diaper. Compared with infants and toddlers, adult body shape has such a larger difference in outer size between the hip and the waist that the diaper can hardly be fastened sufficiently around the waist to fit the body of an adult if used therefor.

Because the absorption core in the crotch region to be applied to the crotch is more voluminous even in the diaper for new bom babies or infants, the expansion of the diaper in the crotch region is increased when the diaper fits the body. If the diaper with the retaining fasteners fastened together is of a cylindrical shape with a uniform inner size, therefore, it is very difficult for such retaining fasteners to fasten sufficiently the diaper around the waist of the wearer or the abdomen thereof.

It is the object of the present invention to provide a disposable diaper which is designed to be securely fastened around the waist of the wearer.

The present invention comprises a disposable diaper comprising a liquid non-permeable back sheet, a liquid permeable top sheet and an absorption core interposed between the two sheets, having a front waist region to be applied to the abdominal area of a wearer, a back waist region to be applied to the dorsal area thereof, and a crotch region to be applied to the crotch, wherein two first retaining members are arranged at an interval in the longitudinal direction on the surface of the front waist region or the back waist region, provided that the direction from the front waist region through the crotch region to the back waist region is defined as the longitudinal direction; wherein two second retaining members to fasten to the first retaining members are arranged at an interval in the longitudinal direction on both the ends of the back waist region to be overlaid over the front waist region or on both the ends of the front waist region to be overlaid over the back waist region; characterised in that the dimension H2 between both the ends of the second retaining members in the longitudinal direction is larger than the dimension H1 between both the ends of the first members, so that the second retaining members can be placed on the first retaining members when the ends of the overlaid regions are arranged obliquely toward the longitudinal direction.

The first retaining members and the second retaining members are similarly arranged at two or more positions at an interval in the longitudinal direction, wherein the aligning pitch P2 of the second retaining members in the longitudinal direction is larger than the aligning pitch P1 of the first retaining members in the longitudinal direction.

When the direction vertical to the longitudinal direction is defined as the crosswise direction, these retaining members are preferably of a rectangular shape with the longer side facing toward the crosswise direction of the diaper.

The second retaining members are for example a retaining sheet comprising a resin and having a plurality of protrusion parts, while the first retaining members are woven fabric or non-woven fabric fastening the protrusion parts. Otherwise, the second retaining members are an adhesive tape, while the first retaining members are a film sheet to which the adhesive tape is bonded.

In accordance with the present invention, the aligning pitch P2 of the second retaining members in the longitudinal direction is larger than the aligning pitch P1 of the first retaining members in the longitudinal direction. When the diaper is worn on . the body, therefore, it is required essentially to arrange the edge parts of the flaps obliquely toward the longitudinal direction of the diaper and the second retaining members are arranged obliquely toward the longitudinal direction to fasten the edge parts onto the first retaining members.

Furthermore, the one retaining members are of a rectangular shape or a band-like shape 'with the longer side toward the crosswise direction, so that the first retaining members are formed of a larger length in the crosswise direction. The width dimension B1 of the first retaining members in the longitudinal direction is sufficiently larger than the width dimension B2 of the second retaining members in the longitudinal direction Then, the angle to slant the edge parts of the flaps of the back waist region can be selected freely at a state of the diaper worn, so that the diaper can be held securely on a human body with any body outline.

When the diaper is worn, the size of the diaper is larger around the hip part while the size of the diaper is smaller around the trunk part (waist part), so that such diaper can readily fit the body. On the waist side, the one retaining members and the other retaining members can be securely retained together, to securely fix the diaper around the waist of the wearer. Accordingly, the diaper hardly shifts in position on the body of the wearer.

Thus, the diaper easily fits the body of an adult. The diaper can be fixed securely around the trunk part (waist part) of new born babies and infants (and toddlers).

Embodiments of the invention are described below with reference to the accompanying drawings, in which:
Fig.1 is a perspective view of the diaper of the present invention at a state when the diaper is worn;
Figs.2 (A) and (B) are developed plane views of the diaper of the present invention; Fig.2(A) is a developed plane view of the front side of the diaper; and Fig.2(B) is a developed plane view of the back side thereof;
Figs.3 (A) and (B) are cross sectional views of Fig.2 or Fig.4 along line III - III; Fig.3(A) depicts the diaper at a flat state without shrinkage; Fig.3(B) depicts the diaper at a state when the elastic members shrink to form front gathering G;
Figs.4(A) and (B) are developed plane views of another example of the diaper of the present invention; Fig.4(A) is a developed plane view of the front side of the diaper; and Fig.4(B) is a developed plane view of the back side of the diaper;
Fig.5 is a developed view of the other example of the diaper of the present invention; and
Fig.6 depicts a protector against incontinence; Fig.6(A) is a developed view; and Fig.6(B) is a perspective view of the protector at a state when it is used.

The present invention will be described in the following examples with reference to drawings hereinbelow.

Diaper 15 is formed of a lamination body, comprising back sheet 16 and top sheet 17, and absorption core 18 interposed between the two sheets. Because the back sheet 16 faces toward the exterior side of a body, the back sheet comprises a resin sheet, liquid non-permeable but air permeable, so as to prevent the oozing of urine outside. Because the top sheet 17 faces toward the skin side of a wearer and is therefore in direct contact to the skin, a material gentle to skin such as liquid permeable, non-woven fabric, is used for the top sheet. For example, use is made of a non-woven fabric with a base weight of 15 g/m² or more to 25 g/m² or less.

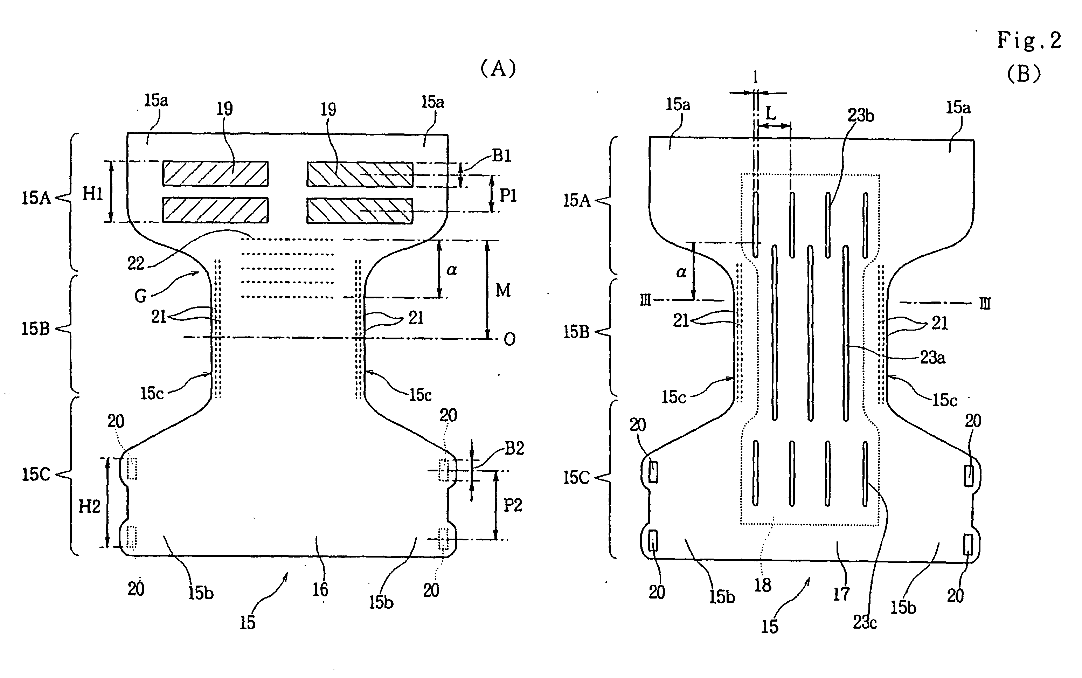

As the absorption core 18, use is made of a crushed pulp or a mixture of a crushed pulp with a super absorbent polymer, capable of well absorbing urine but never causing any unpleasant feeling for the wearer. As the crushed pulp for use as the absorption core 18, generally, use is made of a crushed pulp of a light weight with for example a specific gravity of 100 g/m² or more to 500 g/m² or less. The absorption core 18 is produced by preparing a crushed pulp or super absorbent polymers in a sheet-like form, thereafter preparing the thickness thereof uniformly and cutting the resulting sheet in a sandglass-like form with the width of crotch region 15B more or less narrower. The region surrounded by dotted line in Fig.2(B) is a region where the absorption core 18 is present.

As shown in Figs.2(A) and (B), the front flaps 15a, 15a protruding in the crosswise direction of the diaper are formed in front waist region 15A of the diaper 15. In back waist region 15C, back flaps 15b, 15b are formed, which are present on both the sides of the diaper in the crosswise direction and protrude in the crosswise direction of the back waist region 15C.

The front flaps 15a, 15a and the back flaps 15b, 15b are formed by protruding the back sheet 16 and the top sheet 17 in the crosswise direction of the diaper and bonding the two sheets 16 and 17 together by means of hot-melt adhesive 24, with no absorption core 18 interposed between the back sheet 16 and the top sheet 17. Besides the back sheet 16 and the top sheet 17, alternatively, a resin sheet or a non-woven fabric with a larger base weight is newly cut in a given shape, which is mounted on the back sheet 16 and the top sheet 17 to form front flaps 15a, 15a and back flaps 15b, 15b.

On the surface of the back sheet 16 are fixed first retaining members 19 in the form of retaining fasteners as one example of retaining means, at an interval along the longitudinal direction in the front waist region 15A. Additionally, the second retaining members 20 in the form of retaining fasteners as retaining means are arranged at an interval along the longitudinal direction on the top parts of the back flap 15b (both the sides of the back waist region 15C) . When the diaper 15 is worn, the back waist region 15C is applied to the dorsal area of a wearer while the crotch region 15B is applied to the crotch and the front waist region is applied to the abdominal area. Then, the back flaps 15b, 15b are overlaid on the back sheet 16 in the front waist region 15A to fasten the second retaining members 20 with the first retaining member 19.

As shown in Fig.2(A), the interval P2 between the center lines of the widths of two longitudinally aligned second retaining members 20 in the longitudinal direction is larger than the interval P1 between the center lines of the widths of two longitudinally aligned retaining members 19 in the longitudinal direction. The dimension H2 of the ends of the second retaining members 20, 20 in the longitudinal direction is larger than the whole dimension H1 of the ends of the first retaining members 19, 19 in the longitudinal direction. Furthermore, the width dimension B1 of the first retaining members 19 in the longitudinal direction is sufficiently larger than the width dimension B2 of the retaining members 20 in the longitudinal direction.

In the diaper for adults (aged people), for example, the aligning pitch P1 of the first retaining members 19 is 80 mm, while the aligning pitch P2 of the second retaining members 20 is 135 mm. The ratio P2/P1 is almost 3.1/1.9, preferably 3/2 or more. The dimension H1 is 130 mm while H2 is 165 mm, and the difference is 35 mm. Furthermore, the width dimension B1 is 50 mm, while the width dimension B2 is 30 mm, and B1/B2 is preferably 5/3 or more. Furthermore, the first retaining members 19 are of a rectangular shape or a band-like shape, with the longer side toward the crosswise direction of the diaper, and the longer side thereof is for example 235 mm.

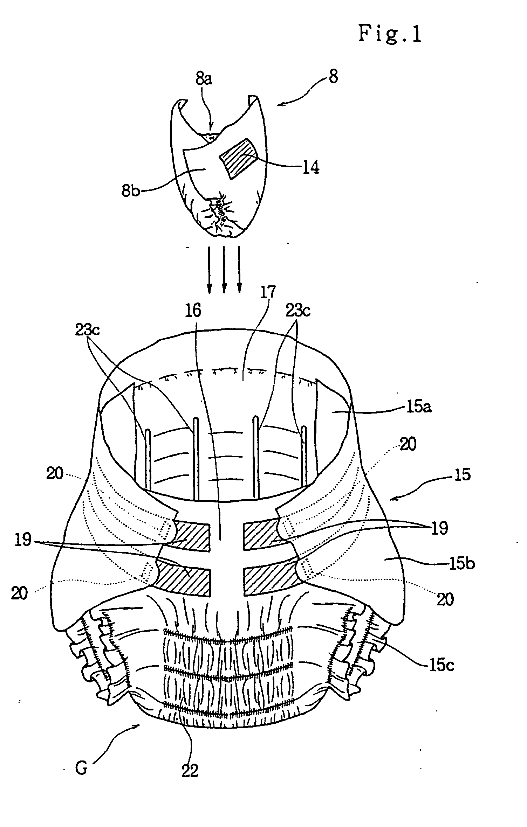

Because the dimension of thefirst retaining members 19 is large in the crosswise direction, the position to attach these retaining members 19 and 20 together can make a certain shift for wearing, depending on the size of the trunk part of a wearer. Additionally, the aligning pitch P2 of the second retaining members 20 is larger than the aligning pitch P1 of the first retaining members 19, while the dimension H2 of the ends of the second retaining members 20 in the longitudinal direction is larger than the dimension H1 of the ends of the first retaining members 19 in the longitudinal direction. At a state of the diaper worn, therefore, the edge part of the back flap 15b is essentially arranged obliquely toward the longitudinal direction, as shown in Fig.1, while the second retaining members 20, 20 face obliquely toward the longitudinal direction to be fastened on the first retaining members 19.

As shown in Fig.1, accordingly, the position to attach the firstretaining members 19 positioned upward in the longitudinal direction of the diaper with the second retaining members 20 is present more inward than the position to attach the first retaining members 19 positioned downward in the longitudinal direction (side of crotch region 15B) with the second retaining members 20. At such state, the second retaining members 20 can be fastened to the first retaining members 19 nearly at the center thereof in the longitudinal direction. Accordingly, at a state of the diaper worn, the hip part of the diaper is large but the trunk part (waist part) thereof is slim, so that the diaper is easy to fit the body and can securely fasten the body around the waist.

In the embodiment, furthermore, the second retaining members 20 are a retaining sheet made of a resin, where a great number of retaining protrusions of a mushroom shape or a hook shape having a retaining head on the top are formed. Alternatively, the first retaining members 19 are woven, tricot-knitted fabric made of a raw material polyester fiber, or non-woven fabric. As the second retaining members 20, an adhesive tape may be arranged satisfactorily instead of the retaining sheet, while instead of woven or non-woven fabric, film may satisfactorily be arranged as the retaining members 19. In this case, adhesive tapes ( second retaining members 20) adhere to the surface of film (first retaining members 19) at a state of the diaper worn.

On both the edge parts of the crotch region 15B of the diaper 15, for example, two flat elastic braids 21, 21 at their elongated state in the longitudinal direction and at an interposed state between the back sheet 16 and the top sheet 17 are bonded to the two sheets. The crotch region 15B shrinks in the longitudinal direction via the flat elastic braids 21, 21 to form crinkles to prepare thigh part gathering 15c. Through the thigh part gathering 15c, the diaper turns into a solid shape with the back sheet 16 protruding. When the diaper 15 is worn, the thigh part gathering 15c appropriately fastens the leg groins of a wearer to prevent the forming of a space between the thigh part and the diaper, so that the diaper can fit the thigh part of the wearer.

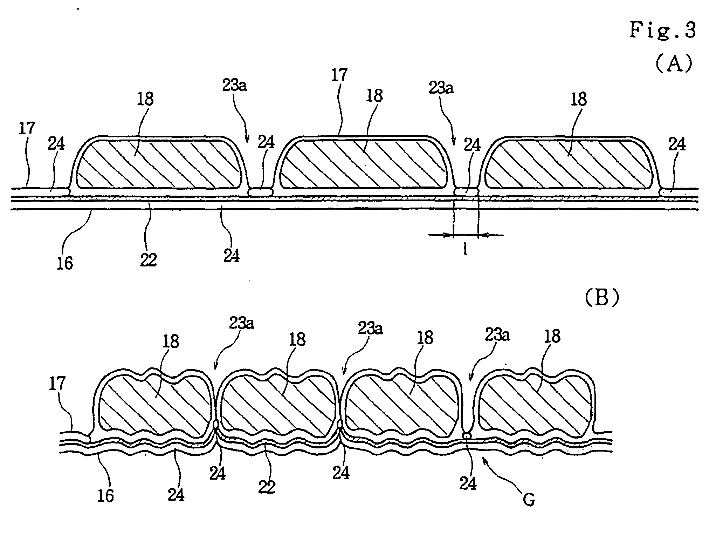

As shown in Fig.2(A), at a part closer to the side of the front waist region from the center part of the crotch region 15B of the diaper 15 in the longitudinal direction, more specifically in region α over the crotch region 15B and the border region between the crotch region 15B and the front waist region 15A, a plurality of elastic members 22 such as flat elastic braid elongating in the crosswise direction of the diaper are arranged at an interval. As shown in Fig. 3 (A), the elastic members 22 are arranged externally on the absorption core 18 and internally on the back sheet 16. Then, the elastic members 22 are bonded and fixed on the back sheet 16 by means of hot-melt adhesive 24 while the elastic members are at an elongated state of a length 1.5-fold to 3-fold, preferably 2-fold to 2.5-fold the initial length. The elastic members 22 and the back sheet 16 are bonded together over the whole length of the elastic members 22 by means of hot-melt adhesive 24. Otherwise, the elastic members 22 and the back sheet 16 are partially bonded together by means of hot-melt adhesive 24. As shown in Fig. 3 (A), furthermore, the elastic members 22, the top sheet 17 and the back sheet 16 are bonded together and fixed by means of the adhesive 24 in the thin thickness parts 23a.

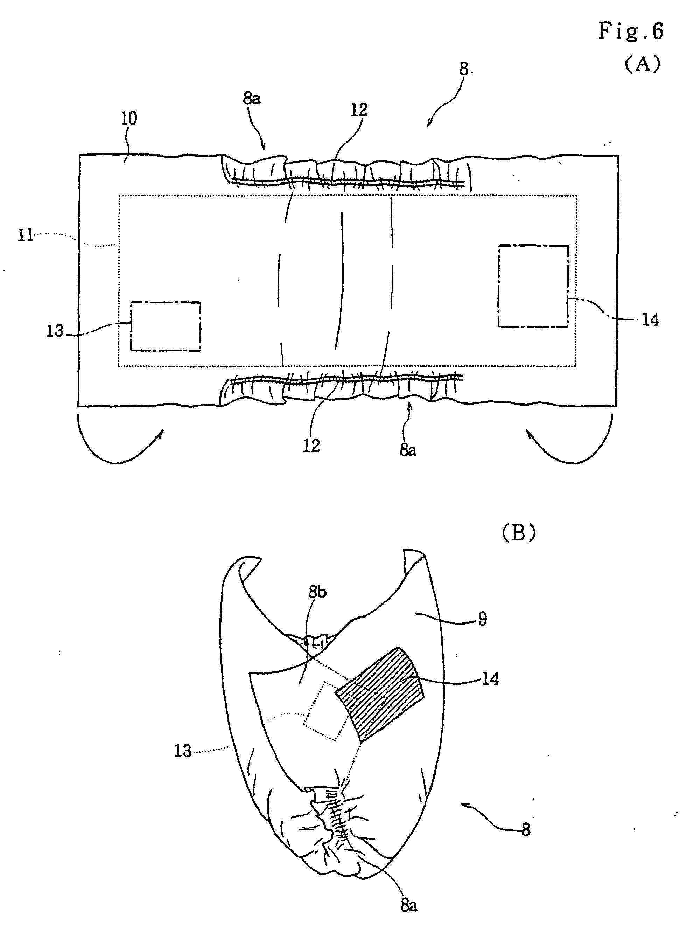

The diaper shrinks in the region **α** in the crosswise direction through the elastic members 22 to form front gathering G. In one example shown in Fig.2, the front gathering G is formed in the crotch region 15B, and the border region between the crotch region 15B and the front waist region 15A. When the diaper is used in combination with protector against incontinence 8 for males as shown in Figs.6(A) and (B), the region α where the elastic members 22 are arranged nearly corresponds to the region where the protector against incontinence 8 is to be mounted. In this case, the distance from the center line O of the crotch region 158 in the crosswise direction to the elastic members 22 arranged at the far end of the front waist region 15A (shown as M in Fig. 2 (A)) is preferably about 250 mm.

As shown in Fig.2(B), a plurality of thin thickness parts 23a, 23b and 23c are formed on nearly the whole surface of the region where the absorption core 18 is presented on the diaper 15. Thin thickness parts 23a are arranged as three lines elongating linearly in the longitudinal direction in the crotch region 15B and at a given interval in the crosswise direction. Additionally, the thin thickness parts 23b and 23c are arranged as four lines elongating linearly in the longitudinal direction and at a given interval in the crosswise direction. The thin thickness parts 23a arranged in the crotch region 15B, and the thin thickness parts 23b and 23c arranged in the front waist region 15A and the back waist region 15C, are independently formed and separated from each other.

The thin thickness parts 23a arranged in the form of the three lines in the crotch region 15B, and the thin thickness parts 23b arranged in the form of the four lines in the front waist region 15A, are alternately aligned of their end parts in the border region between the crotch region 15B and the front waist region 15A. Therefore, in the parts where the thin thickness parts 23a and 23b are alternately overlaid to each other, the thin thickness parts 23a and 23b are alternately aligned in the crosswise direction of the diaper 15. The parts where the thin thickness parts 23a and 23b are arranged alternately are positioned in the region α where the elastic members 22 are arranged to form front gathering G. It is not required that the part where the thin thickness parts 23a and 23b are alternately aligned accurately corresponds to the border region between the crotch region 15B and the front waist region 15A and the part may reside at a part closer to the side of the crotch region 15B in the border region or at a part closer to the side of the front waist region 15A in the border region. The part where the thin thickness parts 23a and 23b are alternately aligned preferably resides in the region α. However, the part may be more or less outside the region α only if the shrinkage force from the elastic members 22 is given to the part.

In the front waist region 15A, furthermore, the one retaining members 19 elongating in a band-like form in the crosswise direction are arranged at an interval in the longitudinal direction. The thin thickness parts 23b formed in the front waist region 15A elongate to be overlaid on the back side of the region where the one retaining members 19 are arranged.

In the embodiment of the present invention as shown in Figs.3 (A) and (B), no absorption core 18 is present in the thin thickness parts 23a, 23b or 23c. At the thin thickness parts 23a, 23b and 23c, the back sheet 16 and the top sheet 17 are directly bonded together by means of hot-melt adhesive 24 or the like. At the thin thickness parts 23a, 23b and 23c where the elastic members 22 are interposed, preferably, the top sheet 17 and the back sheet 16 are bonded to the elastic members 22.

The thin thickness parts 23a, 23b and 23c elongating linearly in the longitudinal direction are arranged on the diaper 15, and in the region α where the thin thickness parts 23a and 23b are formed, elastic shrinkage force from the elastic members 22 is given to the direction vertical to the thin thickness parts.

When the elastic shrinkage force is larger than the rigidity of the absorption core 18, the parts excluding the thin thickness parts are drawn from both the sides in the crosswise direction of the diaper as shown in Fig.3(B), so that the adjacent parts interposing the thin thickness part 23a adhere together. The parts excluding the thin thickness parts shrink more in the crosswise direction, where the absorption core 18 deforms in the shape of protrusion toward the inside of the diaper (the body side of a wearer). The part of the region α of the diaper 15 adheres to the body, with no forming of any space between the diaper 15 and the body. So as to deform the diaper 15 so that the diaper might fit the body, as has been described above, two or more elastic members may satisfactorily be arranged, each elastic member having an elastic force of 10 g or more when the elastic member elongates to a length two-fold the initial length, provided that the rigidity of the absorption core 18 as measured according to JIS P8125 is 3 g · cm or more to 20 g · cm. Then, the elastic shrinkage force from the elastic members 22 exceeds the rigidity of the absorption core 18, so that the parts excluding the thin thickness parts in the absorption core can shrink. If the elastic shrinkage force of the elastic members 22 is too strong, however, the width dimension of the diaper 15 is too small. The upper limit of the elastic force per one elastic member 22 is about 100 g, while the number of the elastic members 22 varies depending on the elastic force from one elastic member. Generally, however, the number of the elastic members is preferably about two to 50.

In the diaper shown in Fig.2(B), furthermore, both of the end of the thin thickness parts 23a on the side of the crotch region 15B and the end of the thin thickness parts 23b on the side of the front waist region 15A are positioned in the region α under the influence of the elastic force from the elastic members 22. Because both the thin thickness parts 23a and 23b are formed separately from each other, additionally, the number of parts divided by the thin thickness parts 23a and 23b in the absorption core 18 is increased in the border region between the crotch region 15B and the front waist region 15A. Therefore, the diaper readily shrinks at the parts in the crosswise direction, so that the diaper hardly expands forward.

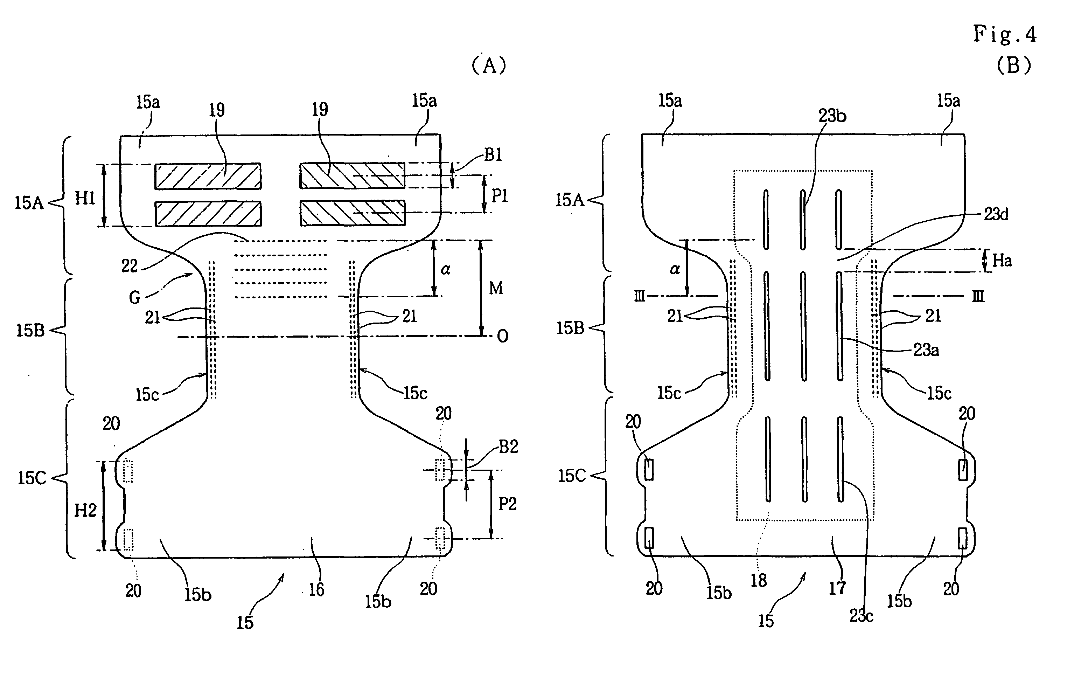

Furthermore, the thin thickness parts 23a and 23b are not necessarily aligned alternately. As long as both the thin thickness parts 23a and 23b are arranged at parts under the influence of the elastic shrinkage force from the elastic members 22, the ends of the two thin thickness parts 23a and 23b may satisfactorily be separated slightly from each other to be present upward and downward in the longitudinal direction, as shown in Fig.4(B). In this case, the crotch region 15B and the front waist region 15A readily shrink in the region α in the crosswise direction, which prevents the forward expansion of the absorption core 18. As shown in Fig.2 (B), however, more satisfactorily, three lines of the thin thickness parts 23a and four lines of the thin thickness parts 23b are alternately arranged in the border region between the crotch region 15B and the front waist region 15A. Because the absorption core 18 is to be divided into eight regions in the crosswise direction at the part, the absorption core 18 under the influence of the shrinkage force from the elastic members 22 is drawn toward the crosswise direction, so that the absorption core more readily shrinks.

Therefore, the diaper 15 fits the body with no forming of any space between the diaper 15 and the body, to prevent the oozing of urine and feces. When the diaper is directly worn, urine is never concentrated at one spot because of the presence of the thin thickness parts 23a of the crotch region 15B, so that urine is readily dispersed over the absorption core in the front waist region 15A and the back waist region 15B.

The thin thickness parts 23a of the crotch region 15B and the thin thickness parts 23b of the front waist region 15A, both receiving the shrinkage force from the elastic members 22, are formed in such a manner that the adjacent thin thickness parts generally have an interval (length L in Fig.2(B)) of about 10 mm or more to 100 mm or less and the width of the thin thickness part 23a or 23b (length 1 in Fig.2(B)) is 1 mm or more to 15 mm or less. Within the range of such numerical figures, the thin thickness parts 23a and 23b are to be formed. In such case, the diaper 15 readily deforms along the body outline of a wearer, to readily disperse urine.

When a male adult wears the diaper 15, protector against incontinence 8 is generally worn as shown in Figs. 6 (A) and (B).

The protector against incontinence 8 is formed of a lamination body in a sheet-like form, comprising outer sheet 9 such as liquid non-permeable resin sheet, inner sheet 10 such as non-woven fabric and absorption core 11 interposed between the two sheets. In the regions at the ends of the lamination body in the longitudinal direction and the width direction, where no absorption core is present, the outer sheet and the inner sheet are bonded together by means of a hot-melt adhesive. At the ends in the width direction, elastic members 12 such as flat elastic braids elongating in the longitudinal direction are interposed and partially bonded at its elongated state between the outer sheet and the inner sheet. The elastic members form gathering 8a.

The protector against incontinence 8 is preliminarily formed as a bag-like shape as shown in Fig.6(B), by molding a sheet-like material shown in Fig.6(A) in a conical shape while the outer sheet 9 faces outward. Then, the protector is worn while the overlaying part 8b is applied to the abdominal area of the body. While the protector against incontinence 8 is worn, the back waist region 15C of the diaper 15 is applied to the dorsal area; the crotch region 15B is applied to the crotch and the front waist region 15A is applied to the abdominal area. By adhesion bonding the adhesive part 14 of the protector against incontinence 8 to the top sheet 17 of the front waist region and then fastening the one retaining members 19 and 20 together, the diaper 15 is worn. Then, the diaper 15 deforms through the thin thickness parts 23a, 23b and 23c along the body outline of the body of the wearer, and furthermore, the region α in the border between the front waist region 15A and the crotch region 15B deforms along the shape of the protector against incontinence 8. Subsequently, the front gathering G as applied to the crotch of the wearer elongates along the outer shape of the protector against incontinence 8, to elastically fit the protector against incontinence 8, whereby the protector against incontinence 8 can be pressed so that the protector might never be free from the body of the wearer. Thus, the protector against incontinence 8 does not make any shift in position, so that the protector against incontinence 8 effectively absorbs urine.

When the diaper 15 is used for females, additionally, the protector against incontinence 8 is overlaid on the top sheet 17 of the crotch region 15B of the diaper 15 while the protector against incontinence 8 remains as the sheet-like form as shown in Fig. 6(A), and at such state, the diaper 15 is worn. The front gathering G is in contact to the protector against incontinence 8, so that the protector against incontinence 8 can be retained at a stable state by means of the front gathering G. When the protector against incontinence 8 is used for females, preferably, elastic members 22 are arranged more closely to the side of the center line O as shown in Fig.2(A).

When the protector against incontinence 8 is used in such manner, the diaper worn expands between the crotch and hip parts. As has been described above, however, the second retaining members 20, 20 at their oblique state in the longitudinal direction are fastened to the first retaining members 19, 19 in the diaper. Therefore, even the diaper with an expansion formed as described above can have a slimmer waist part to securely fasten the retaining members 19, 20 around the waist of the wearer.

When the diaper 15 is singly worn with no use of any protector against incontinence, the front gathering G deforms along the shape of the crotch of a wearer to elastically fit the crotch. Thus, no space is formed between the body of the wearer and the diaper 15, so that the side oozing of urine and feces from the diaper can be prevented, whereby urine and feces can be retained securely on the diaper 15. Through the thin thickness parts 23a, 23b and 23c, urine can be dispersed not only over the crotch region, without any concentration, so that the absorption potency of the diaper 15 can be improved.

As shown in Figs.2(A) and (B), furthermore, the thin thickness parts 23b formed in the front waist region 15A elongate to a position where the thin thickness parts 23b are overlaid over the one retaining members 19. When the back flaps 15b, 15b are overlaid on the front waist region 15A to fasten the retaining members 19, 20 together, the surface of the front waist region 15A readily deforms. If the front gathering G is formed around the part where the one retaining members 19 are arranged owing to the presence of the elastic members 22 as shown in Fig. 2 (A), in particular, the shrinkage force of the elastic members 22 acts on a part of the first retaining members 19 so that the first retaining members 19 readily receive the shrinkage force in the crosswise direction.

At a part where the first retaining members 19 are arranged the width of the thin thickness part 23b is narrowed similarly as shown in Fig.3(A), because of the shrinkage force, but at a part where the absorption core is present between the thin thickness part 23b and the thin thickness part 23b, the thin thickness parts 23b slightly shrink in the crosswise direction but the surface is still flat. At the first retaining members 19, therefore, the part between the thin thickness parts 23b, 23b is at a flat state and the area of the flat part is relatively large. Hence, the second retaining members 20 can be bonded to the flat part of the first retaining members 19. Thus, the retaining strength between the retaining members 19, 20 can be kept large.

As has been described above, in Figs. 4 (A) and (B), the end parts of the thin thickness parts 23a in the crotch region 15B and of the thin thickness parts 23b in the front waist region 15A are more or less separated from each other in the longitudinal direction, and the region of a given width Ha between the thin thickness parts 23a and 23b in the longitudinal direction corresponds to thick thickness part 23d with no thin thickness part formed thereon. The thick thickness part 23d is positioned in the region α where the elastic members 22 are arranged to form front gathering G.

As shown in Fig. 3(B), due to the shrinkage of the elastic members 22 in the crosswise direction, recesses are formed at the part where the thin thickness parts 23a or 23b are formed. Therefore, recess is formed on the top sheet 17, to make the irregularity of the top sheet 17 more prominent. When the thick thickness part 23d is arranged, however, the irregularity of the top sheet 17 is slightly reduced at the thick thickness part 23d, to consequently enlarge the relatively flat area. When the protector against incontinence 8 is positioned inside the front gathering G and the adhesive part 14 adheres to the thick thickness part 23d, the area where the adhesive part 14 and the top sheet 17 face to each other can be enlarged because no thin thickness part is present. Thus, the adhesion strength of the protector against incontinence 8 to the diaper can be enhanced, whereby the shift of the protector against incontinence 8 can be prevented.

As has been described above, additionally, the thin thickness parts 23a and 23b are present in front of and behind the thick thickness part 23d. Thus, it never occurs that the diaper 15 hardly shrinks due to the presence of the thick thickness part 23d.

The thick thickness part 23d is formed at a part to be spotted with urine. Because a greater volume of the absorption core 18 is present at the thick thickness part 23d, the reduction of the absorbed urine in volume can be prevented.

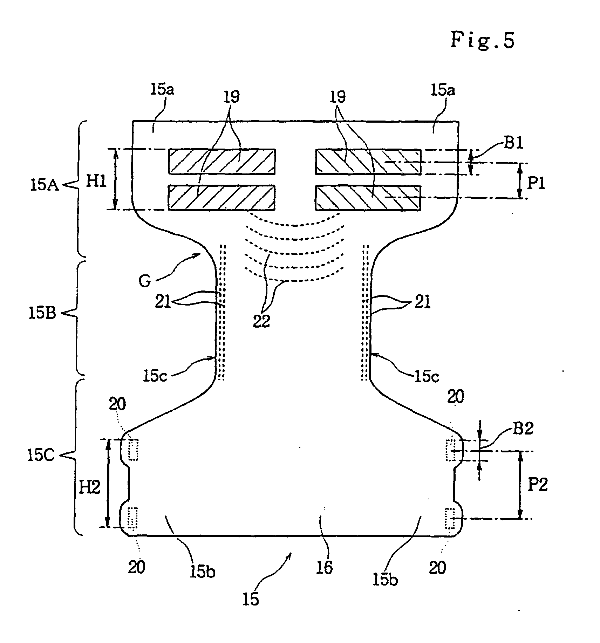

Fig.5 depicts another embodiment of the diaper of the present invention, which is a view of the diaper 15 at a developed state, as viewed from the outside. In the embodiment, elastic members 22 are arranged with a curve protruding toward the center direction of the crotch region 15B of the diaper 15, from both the ends of the diaper 15 to the center thereof. If the elastic members 22a are arranged in such fashion, the diaper 15 deforms more fittingly along the protector against incontinence 8 and the crotch shape of a wearer, so that the diaper 15 can fit the body more.

Even in Fig.5, furthermore, the aligning pitch P2 of the second retaining members 20 in the longitudinal direction is larger than the aligning pitch P1 of the first retaining members 19 in the longitudinal direction. Additionally, the dimension H2 between the end parts of the second retaining members 20 is larger than the dimension H1 between the end parts of the first retaining members 19. Still further, the width dimension B1 of the firstretaining members 19 in the longitudinal direction is sufficiently larger than the width dimension B2 of the second retaining members 20 in the longitudinal direction.

When the diaper 15 is worn on the body, the trunk part of the diaper 15 can essentially be slimmed to securely fasten the diaper 15 around the waist part of wearer.

Even in Fig.5, the thin thickness parts 23b are formed in a region to be overlaid on the one retaining members 19 in the front waist region 15A.

The present invention has been described in the examples of an open type diaper, but the effect of arranging the thin thickness parts 23a, 23b and 23c and the effect of forming the front gathering G, in accordance with the present invention, are also exerted even if the thin thickness parts are formed in a brief-type diaper. Such thin thickness parts may satisfactorily be formed in diapers not only for adults but also for infants (and toddlers).

As has been described in detail insofar, in the diaper of the present invention, elastic members are arranged in the front waist region or the border region between the crotch region and the front waist region to form gathering. In the region where the gathering is formed, the.crotch of a wearer can be pressed elastically. Thus, no space is formed between the diaper and the crotch of a wearer, so that the diaper can fit the crotch.

Because the thin thickness parts elongating in the longitudinal direction of the diaper are formed, the diaper can readily deform along the body outline of the wearer owing to the presence of the thin thickness parts. And the absorption core shrinks in the crosswise direction to effectively prevent the expansion of the crotch region in a bag-like form. Furthermore, the thin thickness parts formed in the crotch region elongate into the region between the thin thickness parts in the front waist region and in the border region between the front waist region, and the crotch region, the thin thickness parts in the front waist region and the thin thickness parts in the crotch region are alternately arranged. Owing to the presence of the gathering formed in the region the diaper shrinks in the crosswise direction but hardly expands forward, so that the diaper deforms into a shape adjusting to the crotch of a wearer. Through the gathering, the diaper can fit the crotch of the wearer in such manner.

When the diaper is used in combination with a protector against incontinence, therefore, the diaper does not shift the protector against incontinence in position to securely retain the protector against incontinence and absorb urine effectively owing to the presence of the protector against incontinence. Even when the diaper is used singly with no use of any protector against incontinence, the diaper still can retain excretion such as urine and feces securely, without causing any side oozing thereof.

Because the elastic members are arranged only at the part where the protector against incontinence is mounted, additionally, the parts excluding the aforementioned part can touch skin at the natural state, which gives pleasant feeling to the whole body when the diaper is worn.

By determining relatively the positions of the first retaining members and the second retaining members, additionally, the diaper can be made broad around the hip part and be made slim around the trunk part when the diaper is worn. Thus, the diaper can be fastened securely around the trunk part of a wearer, whereby the positional shift of the diaper hardly occurs. Even if the diaper expands in the crotch or at the hip part, the diaper can securely fasten the trunk part of the wearer so that the diaper can readily fit the body shape of the wearer.

## Claims

1. A disposable diaper (15) comprising a liquid non-permeable back sheet (16), a liquid permeable top sheet (17) and an absorption core (18) interposed between the two sheets, having a front waist region (15A) to be applied to the abdominal area of a wearer, a back waist region (15C) to be applied to the dorsal area thereof, and a crotch region to be applied to the crotch (15B),
wherein two first retaining members (19) are arranged at an interval in the longitudinal direction on the surface of the front waist region or the back waist region, provided that the direction from the front waist region through the crotch region to the back waist region is defined as the longitudinal direction; wherein two second retaining members (20) to fasten to the first retaining members are arranged at an interval in the longitudinal direction on both the ends of the back waist region to be overlaid over the front waist region or on both the ends of the front waist region to be overlaid over the back waist region;
**characterised in that** the dimension H2 between both the ends of the second retaining members (20) in the longitudinal direction is larger than the dimension H1 between both the ends of the first members (19), so that the second retaining members (20) can be placed on the first retaining members (19) when the ends of the overlaid regions are arranged obliquely toward the longitudinal direction.

2. A disposable diaper according to Claim 1,
wherein the aligning pitch P2 of the second retaining members (20) arranged at an interval in the longitudinal direction is larger than the aligning pitch P of the first retaining members (19) arranged at an interval in the longitudinal direction.

3. A disposable diaper according to Claim 2,
wherein the width dimension B1 of the first retaining members (19) in the longitudinal direction is larger than the width dimension B2 of the second retaining members (20) in the longitudinal direction.

4. A disposable diaper according to Claim 1,
wherein the first retaining members (19) are of a rectangular shape with the longer side facing toward the crosswise direction of the diaper, provided that the direction vertical to the longitudinal direction is defined as the crosswise direction.

5. A disposable diaper according to Claim 1,
wherein flaps (15a;15b) are formed on both the sides of the front waist region or the back waist region and the second retaining members (20) are formed on the flaps.

6. A disposable diaper according to Claim 1,
wherein the first retaining members (19) and the second retaining members (20) can be fastened together so that the inner diameter of the part worn around the body waist might be smaller than the inner diameter of the part worn around the hip at a state when the diaper is worn to overlay the back waist region (15C) over the front waist region (15A).

7. A disposable diaper according to Claim 1,
wherein the first retaining members (19) are a woven fabric sheet or a non-woven fabric sheet to be fastened with fastening protrusions, and
wherein the second retaining members (20) are a resin-made fastening sheet with a plurality of fastening protrusions.

8. A disposable diaper according to Claim 1,
wherein the first retaining members (19) are a resin film and the second retaining members (20) are an adhesive tape bonding to the one retaining members.

## Patentansprüche

1. Eine Wegwerfwindel (15), die eine flüssigkeitsundurchlässige Rückschicht (16), eine flüssigkeitsdurchlässige Deckschicht (17) und einen zwischen den zwei Schichten angeordneten Absorptionskern (18) umfasst, und eine vordere Taillenregion (15A), die um den Bauchbereich einer Trägerperson anzubringen ist, eine hintere Taillenregion (15C), die an den Rückenbereich davon anzubringen ist, und eine Schrittregion, die im Schritt (15B) anzubringen ist, aufweist,
worin zwei erste Befestigungselemente (19) mit Abstand in der Längsrichtung auf der Oberfläche der vorderen Taillenregion oder der hinteren Taillenregion angeordnet sind, vorausgesetzt, dass die Richtung ab der vorderen Taillenregion durch die Schrittregion zur hinteren Taillenregion als die Längsrichtung definiert ist; worin zwei zweite Befestigungselemente (20) zur Befestigung an die ersten Befestigungselemente mit Abstand in der Längsrichtung an beiden der Enden der hinteren Taillenregion angeordnet sind, die über die vordere Taillenregion oder an beiden der Enden der vorderen Taillenregion über die hintere Taillenregion überlagert werden sollen;
**dadurch gekennzeichnet, dass** die Dimension H2 zwischen beiden der Enden der zweiten Befestigungselemente (20) in der Längsrichtung größer als die Dimension HI zwischen beiden Enden der ersten Elemente (19) ist, so dass die zweiten Befestigungselemente (20) auf die ersten Befestigungselemente (19) platziert werden können, wenn die Enden der wenn die Enden der überlagerten Regionen schräg in Richtung der Längsrichtung angeordnet sind.

2. Eine Wegwerfwindel nach Anspruch 1,
worin die Ausrichtteilung P2 der zweiten Befestigungselemente (20), die mit einem Abstand in der Längsrichtung angeordnet sind, größer als die Ausrichtteilung P1 der ersten Befestigungselemente (19) ist, die mit einem Abstand in der Längsrichtung angeordnet sind.

3. Eine Wegwerfwindel nach Anspruch 2,
worin die Breitendimension B1 der ersten Befestigungselemente (19) in der Längsrichtung größer als die Breitendimension B2 der zweiten Befestigungselemente (20) in der Längsrichtung ist.

4. Eine Wegwerfwindel nach Anspruch 1,
worin die ersten Befestigungselemente (19) einer rechteckigen Form sind, wobei die längere Seite in Richtung der Querrichtung der Windel zeigt, vorausgesetzt, dass die zur Längsrichtung vertikale Richtung als die Querrichtung definiert ist.

5. Eine Wegwerfwindel nach Anspruch 1,
worin Klappen (15a;15b) auf beiden der Seiten der vorderen Taillenregion oder der hinteren Taillenregion geformt sind und die zweiten Befestigungselemente (20) auf den Klappen geformt sind.

6. Eine Wegwerfwindel nach Anspruch 1,
worin die ersten Befestigungselemente (19) und die zweiten Befestigungselemente (20) so miteinander verbunden werden können, dass der Innendurchmesser des um die Körpertaille getragenen Teils kleiner als der Innendurchmesser des um die Hüfte getragenen Teils in einem Zustand sein könnte, wenn die Wegwerfwindel getragen wird, um die hintere Taillenregion (15C) der vorderen Taillenregion (15A) zu überlagern.

7. Eine Wegwerfwindel nach Anspruch 1,
worin die ersten Befestigungselemente (19) einer Webstoffschicht oder einer Vliesstoffschicht sind, die mit Befestigungsvorsprüngen befestigt werden sollen, and
worin die zweiten Befestigungselemente (20) eine aus Harz hergestellte Befestigungsschicht mit einer Vielheit von Befestigungsvorsprüngen sind.

8. Eine Wegwerfwindel nach Anspruch 1,
worin die ersten Befestigungselemente (19) eine Harzfolie sind und die zweiten Befestigungselemente (20) ein Klebeband zum Kleben an die ersten Befestigungselemente sind.

## Revendications

1. Couche jetable (15) comportant une feuille arrière imperméable aux liquides (16), une feuille supérieure perméable aux liquides (17) et un coeur absorbant (18) placé entre les deux feuilles, ayant une région avant de la taille (15A) devant être appliquée sur la région abdominale de la personne qui la porte, une région arrière de la taille (15C) devant être appliquée sur la région dorsale de celle-ci et une région d'entre-jambes (15B) devant être appliquée sur l'entre-jambes,
dans laquelle deux premiers éléments de retenue (19) sont disposés avec un intervalle dans la direction longitudinale sur la surface de la région avant de la taille ou de la région arrière de la taille, en supposant que la direction allant de la région avant de la taille, passant par la région de l'entre-jambes vers la région arrière de la taille, est définie comme la direction longitudinale ; dans laquelle deux deuxièmes éléments de retenue (20) à attacher aux premiers éléments de retenue sont disposés avec un intervalle dans la direction longitudinale sur les deux extrémités de la région arrière de la taille devant être placées au-dessus de la région avant de la taille, ou sur les deux extrémités de la région avant de la taille devant être placées au-dessus de la région arrière de la taille ;
**caractérisée en ce que** la distance H2 entre les deux extrémités des deuxièmes éléments de retenue (20) dans la direction longitudinale est plus grande que la distance H1 entre les deux extrémités des premiers éléments (19), de façon que les deuxièmes éléments de retenue (20) puissent être placés sur les premiers éléments de retenue (19) quand les extrémités des régions superposées sont disposées de manière oblique dans la direction longitudinale.

2. Couche jetable selon la revendication 1,
dans laquelle le pas d'alignement P2 des deuxièmes éléments de retenue (20) espacés d'un intervalle dans la direction longitudinale est plus grand que le pas d'alignement P1 des premiers éléments de retenue (19) espacés d'un intervalle dans la direction longitudinale.

3. Couche jetable selon la revendication 2,
dans laquelle la largeur B1 des premiers éléments de retenue (19) dans la direction longitudinale est plus grande que la largeur B2 des deuxièmes éléments de retenue (20) dans la direction longitudinale.

4. Couche jetable selon la revendication 1,
dans laquelle les premiers éléments de retenue (19) sont de forme rectangulaire avec les côtés les plus longs en face de la direction transversale de la couche, en supposant que la direction verticale par rapport à la direction longitudinale est définie comme la direction transversale.

5. Couche jetable selon la revendication 1,
dans laquelle des rabats (15a, 15b) sont formés sur les deux côtés de la région avant de la taille ou de la région arrière de la taille, et les deuxièmes éléments de retenue (20) sont formés sur les rabats.

6. Couche jetable selon la revendication 1,
dans laquelle les premiers éléments de retenue (19) et les deuxièmes éléments de retenue (20) peuvent être attachés ensemble de telle façon que le diamètre intérieur de la partie située autour de la taille puisse être plus petit que le diamètre intérieur de la partie située autour de la hanche, de sorte que, lorsque la couche est portée, la région arrière de la taille (15C) recouvre la région avant de la taille (15A).

7. Couche jetable selon la revendication 1,
dans laquelle les premiers éléments de retenue (19) sont une feuille de matériau tissé ou une feuille de matériau non-tissé à attacher avec des saillies d'attache, et
dans laquelle les deuxièmes éléments de retenue (20) sont une feuille d'attache en résine avec une pluralité de saillies d'attache.

8. Couche jetable selon la revendication 1,
dans laquelle les premiers éléments de retenue (19) sont un film de résine et les deuxièmes éléments de retenue (20) sont un ruban adhésif collant sur les éléments de retenue.
